Europäisches Patentamt

European Patent Office

Office européen des brevets

(19)

(11) Numéro de publication : **0 125 951 B1**

(12) **FASCICULE DE BREVET EUROPÉEN**

(45) Date de publication du fascicule du brevet :
08.07.87

(51) Int. Cl.4 : **C 07 C 29/136, C 07 C 31/38**

(21) Numéro de dépôt : **84400714.6**

(22) Date de dépôt : **11.04.84**

(54) **Procédé de préparation de trifluoroéthanol.**

(30) Priorité : **21.04.83 FR 8306530**

(43) Date de publication de la demande :
**21.11.84 Bulletin 84/47**

(45) Mention de la délivrance du brevet :
**08.07.87 Bulletin 87/28**

(84) Etats contractants désignés :
**AT BE CH DE FR GB IT LI LU NL SE**

(56) Documents cités :
**US-A- 4 232 170**
**US-A- 4 255 594**
**US-A- 4 273 947**
**THE JOURNAL OF ORGANIC CHEMISTRY, vol. 44, no. 18, 31 août 1979, pages 3268-3269, American Chemical Society, US; M. NOVOTNY: "Catalytic reduction of trifluoroacetic acid under mild conditions"**
**CHEMICAL ABSTRACTS, vol. 98, 1983, page 164, no. 182380c, Columbus, Ohio, US; M. NOVOTNY et al.: "Synthesis of C2-oxygenated chemicals from methanol"**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Cordier, Georges**
**50, Chemin des Hermières**
**F-69340 Francheville (FR)**

(74) Mandataire : **Cazes, Jean-Marie et al**
**RHONE-POULENC INTERSERVICES Service Brevets Chimie 25, quai Paul Doumer**
**F-92408 Courbevoie Cédex (FR)**

## Description

La présente invention concerne un procédé de préparation de trifluoroéthanol ; elle concerne plus particulièrement un procédé de préparation de trifluoroéthanol à partir d'acide trifluoroacétique.

On connaît dans l'art antérieur la demande de brevet français 70.31913 publiée sous le n° 2.060.357 qui décrit la préparation de dihydro-1,1 perfluoroalcanols répondant à la formule générale $C_nF_{2n+1}CH_2OH$ dans laquelle $n > 3$ par hydrogénation d'acides ou d'esters de formule $C_nF_{2n+1}COOR$ où R représente un atome d'hydrogène ou un radical alkyle et où $n > 3$ en présence d'un catalyseur au ruthénium.

Selon ce procédé on effectue l'hydrogénation en présence de 4 à 1 000 % en poids d'eau par rapport à l'acide ou à l'ester et sous température et pression d'hydrogène élevées. La température est comprise entre 80 et 240 °C et la pression d'hydrogène initiale varie entre 5 et 700 kgp/cm².

Ce procédé ne prévoit pas la possibilité de mettre en œuvre l'acide trifluoroacétique (n = 1) pour obtenir le trifluoroéthanol.

La préparation de ce même trifluoroéthanol n'est pas non plus envisagée dans le brevet des Etats-Unis d'Amérique 2.862.977 qui décrit la préparation de dihydro-1,1 perfluoroalkylalcools de formule $C_nF_{2n+1}CH_2OH$ (n > 1) par hydrogénation de l'acide correspondant sous une pression d'au moins 1 000 p. s. i. (70 bars) et à une température supérieure à 150 °C en présence d'un catalyseur au ruthénium.

On connaît encore le brevet des Etats-Unis d'Amérique 4.273.947 qui décrit un procédé pour hydrogéner l'acide trifluoroacétique ($CF_3COOH$) selon lequel on met en contact cet acide en phase liquide avec de l'hydrogène en présence d'un catalyseur supporté ou non supporté à base de rhodium ou d'iridium sous une pression de 5 à 15 atmosphères et à une température de 50 à 150 °C.

Les exemples 1 à 9 de ce brevet américain montrent que dans une enceinte réactionnelle dont le volume est de 0,206 litre on met en œuvre des quantités d'acide trifluoroacétique très faibles (de 1,9 à 3,31 g). Cela correspond à un rapport volume de liquide mis en œuvre au volume de l'enceinte réactionnelle compris entre environ 0,007 et environ 0,015 (l'exemple 10 concerne un procédé en phase vapeur). Il est clair pour l'homme de l'art que ces conditions sont incompatibles avec une exploitation industrielle rentable.

En étudiant les procédés de l'art antérieur, ci-dessus analysés, la demanderesse a constaté que dans le cas de l'hydrogénation de l'acide trifluoroacétique il se développe des réactions secondaires d'hydrogénolyse qui donnent naissance à des produits gazeux comme HF, $CF_3$—$CH_3$, $CH_3$—$CH_3$ et $CH_4$ qui, s'accumulant dans le réacteur, bloquent la réaction d'hydrogénation. Ces réactions secondaires existent aussi dans le cas des acides ayant un nombre d'atomes de carbone plus élevé mais, dans ce cas, les produits secondaires ont une tension de vapeur beaucoup plus faible et n'affectent pas aussi négativement la réaction d'hydrogénation. La demanderesse a de plus constaté que ces réactions secondaires étaient plus importantes dans le cas d'un catalyseur au ruthénium que dans le cas d'un catalyseur au rhodium.

La demanderesse a découvert, d'une part, que ces réactions secondaires se développent lors de la réaction d'hydrogénation et, d'autre part, qu'elles diminuent, du fait de la production de ces inertes gazeux à pression totale constante, la pression partielle d'hydrogène jusqu'à l'arrêt de la réaction principale.

La demanderesse a de plus constaté que pour pouvoir hydrogéner de l'acide trifluoroacétique en présence d'un catalyseur au ruthénium, il faudrait se placer sous forte pression et dans un grand volume réactionnel de façon à ce que les produits secondaires gazeux n'affectent pas la réaction. Ces deux conditions altèrent très fortement l'économie d'un procédé industriel.

En présence d'un catalyseur au rhodium, il faut opérer soit sous forte pression, soit sous basse pression mais alors dans un volume réactionnel important tel que souligné ci-dessus lors de l'analyse du procédé de l'art antérieur décrit dans le brevet des Etats-Unis d'Amérique 4.273.947. Cette condition au niveau du réacteur rend ce procédé incompatible avec une échelle industrielle où généralement on opère avec un rapport du volume de liquide mis en œuvre au volume total du réacteur supérieur à 0,5 environ.

La demanderesse a maintenant découvert un procédé permettant à la fois d'opérer sous faible pression et dans des conditions réactionnelles volumiques satisfaisantes.

L'invention a donc pour objet un procédé de préparation de trifluoroéthanol par hydrogénation en phase liquide d'acide trifluoroacétique caractérisé en ce qu'on opère en présence d'un catalyseur à base de ruthénium, rhodium, iridium ou platine, sous une pression totale comprise entre 5 et 50 bars, avec un rapport du volume de la phase liquide mise en œuvre dans le réacteur au volume total du réacteur supérieur à 1/2 environ et en ce que l'on maintient pendant la réaction une pression partielle d'hydrogène supérieure à 1 bar absolu en laissant échapper en continu au cours de la réaction une partie de la phase gazeuse présente dans le réacteur.

Comme cela résulte de ce qui précède, l'invention est plus particulièrement intéressante pour opérer dans un domaine de pression totale allant de 5 à 30 bars.

Selon un autre mode préférentiel de mise en œuvre de l'invention, on opère avec un chargement du réacteur correspondant à un rapport du volume de la phase liquide au volume total du réacteur compris entre 0,5 et 0,8.

Selon un autre mode préférentiel de l'invention on maintient la pression partielle d'hydrogène à une valeur supérieure à 5 bars. Il est clair, maintenant, pour l'homme de l'art que plus la pression totale sera faible et plus la pression partielle d'hydrogène sera élevée, plus la réaction sera rapide. Un compromis entre ces deux tendances sera aisément trouvé par l'homme de l'art compte-tenu des contraintes technologiques et économiques face auxquelles il sera placé.

Les catalyseurs mis en œuvre dans le cadre du procédé selon l'invention peuvent être sous forme métallique proprement dite, sous forme d'un oxyde ou d'un sel ou un mélange d'entre eux. Ils peuvent être, de plus, déposés sur support ou pas.

On peut utiliser tout support inerte dans les conditions de la réaction comme le charbon, la silice ou l'alumine. On préférera tout particulièrement utiliser le charbon.

La quantité de métal déposée sur le support est de préférence comprise entre 0,1 et 20 % en poids, plus préférentiellement entre 1 et 10 %.

On peut citer comme exemples de catalyseurs : les ruthénium, rhodium, iridium ou platine sous forme métallique, leurs oxydes, hydroxydes et sels d'acides (halogénures, nitrates, sulfates et acétates) et des mélanges d'entre eux éventuellement déposés sur charbon ou silice.

Selon un mode de réalisation tout particulièrement préféré de réalisation on utilise du rhodium métallique déposé à 5 % sur charbon.

On opère de préférence à une température comprise entre 20 et 200°. Encore plus préférentiellement la température est comprise entre 70 et 150 °C.

La quantité de catalyseur (exprimée en % du métal) mise en œuvre est de préférence inférieure à 10 % en poids par rapport à l'acide trifluoroacétique. Encore plus préférentiellement, on utilise moins de 2 % de catalyseur.

La réaction a lieu, de préférence, en présence d'un solvant. Toutefois, il n'est pas exclu d'opérer sans solvant. On peut utiliser de l'eau ou un composé organique inerte (alcane par exemple). L'eau facilite la mise en œuvre de catalyseurs qui généralement sont vendus humides.

D'autres avantages et caractéristiques de l'invention apparaîtront à la lecture des exemples qui suivent :

Exemple 1

Dans un autoclave en Hastelloy $B_2$ résistant à la corrosion, de volume utile 300 ml équipé d'une agitation par turbine on charge :

25 g d'eau distillée

3,9 g d'un catalyseur au Rhodium déposé à 5 % sur un charbon de grande surface spécifique (> 900 m²/g)

298 g soit 2,61 moles d'acide trifluoroacétique.

Après élimination de l'air par un gaz inerte tel que l'azote, puis remplacement de ce dernier par de l'hydrogène, on porte la pression du réacteur à 28 bars, toujours à l'aide d'hydrogène et on chauffe à 90 °C. La réaction s'amorce pendant la montée en température et de ce fait la consommation d'hydrogène compense l'augmentation de pression liée à la dilatation des gaz. On alimente alors en continu l'hydrogène nécessaire à la réaction en maintenant la pression totale à 28 bars.

Lorsque la température atteint 90 °C, tout en maintenant par l'alimentation d'hydrogène la pression totale à 28 bars, on procède à une purge continue du ciel gazeux (après qu'il ait été refroidi à 13-15 °C dans un condenseur).

Cette purge permet l'élimination de composés incondensables à cette température et sous-produits de la réaction (méthane, éthane, trifluoroéthane essentiellement).

Le débit de la purge est réglé à 2,7 lh⁻¹ (TPN) ce qui représente compte tenu de la durée de la réaction environ 10 % de la quantité totale d'hydrogène nécessaire à la réaction.

Celle-ci stoppe après 4 heures et 10 minutes.

Après refroidissement et dégazage de l'enceinte l'analyse de la masse réactionnelle par CPV montre que le taux de transformation de l'acide trifluoroacétique s'élève à 97,5 % ; le rendement en trifluoroéthanol s'élève à 97,5 % et celui des sous-produits volatils définis précédemment s'élève à 2,5 %.

La solution est ensuite neutralisée par la quantité de soude adéquate, filtrée et soumise à une distillation sous pression atmosphérique pour séparer le TFE de l'eau et des sels minéraux résultant de la neutralisation.

Le catalyseur ainsi utilisé a pu être recyclé 3 fois sans perte d'activité apparente.

Exemple 2

Dans un autoclave en acier émaillé de 4 litres de volume utile, on charge :

37,5 g d'un catalyseur au Rhodium déposé à 5 % sur un charbon de grande surface spécifique

300 g d'eau distillée

2 850 g soit 25 moles d'acide trifluoroacétique.

On opère ensuite comme dans l'exemple 1 sauf que la pression totale de l'enceinte est maintenue à 10 bars.

Lorsque la température atteint 90 °C et pendant toute la durée de la réaction, on effectue une purge continue du ciel gazeux préalablement refroidi à 13-15 °C dans un condenseur, tout en maintenant la pression totale de l'enceinte à 10 bars.

Le débit (TPN) de cette purge est de 5,5 lh⁻¹, ce qui représente 11 % environ de la quantité d'hydrogène totale nécessaire à la transformation du TFA en TFE.

La réaction stoppe après 22 heures et 30 minutes. On répète alors les analyses et les opérations décrites dans l'exemple 1.

Le taux de transformation du TFA est de 99,3 % ; le rendement (RT) en TFE est de 95,7 %, le rendement en trifluoroéthane est de 4 % et on ne trouve que des traces d'éthane et de méthane.

Exemple 3

On répète l'exemple 1 en chargeant :
8,8 g du catalyseur Rhodium à 5 % décrit précédemment
25 g d'eau
251 g d'acide trifluoroacétique (TFA) soit 2,2 moles.

On effectue la réaction à 90 °C sous 10 bars de pression totale et on purge en continu 0,25 $lh^{-1}$ (TPN) soit une quantité totale de ciel gazeux correspondant à 1 % environ de la quantité totale d'hydrogène nécessaire à l'accomplissement de la réaction.

La réaction stoppe après 4 heures 10 minutes.

Le taux de transformation de l'acide trifluoroacétique est de 100 %.

Le rendement (RT) en trifluoroéthanol (TFE) est de 99 %. Il s'est formé 1 % de composés résultant de l'hydrogénolyse ($CF_3CH_3$, $C_2H_6$, $CH_4$).

Exemple 4

On opère dans le même réacteur que celui décrit à l'exemple 1. On y charge :
26 g d'un catalyseur à 5 % de ruthénium déposé sur un charbon de grande surface spécifique (> 900 $m^2/g$)
70 g d'eau
179 g (1,57 mole) de TFA.

La réaction est conduite comme dans l'exemple 1 à 90 °C sous 28 bars de pression totale.

Le débit de purge est fixé à 1,0 $lh^{-1}$ (TPN), soit 16 % environ de la quantité d'hydrogène nécessaire à la réaction.

La réaction stoppe après 11 heures et 30 minutes.

Le taux de conversion du TFA est de 96,2 %.

Le rendement (RT) en TFE est de 93,5 % ; celui de $CF_3CH_3$ de 6,4 %. Il y a en outre 0,3 % de $CH_4$ et 0,06 % d'éthane.

Exemple 5

Dans un autoclave de 40 ml résistant à la corrosion de l'acide trifluoroacétique on charge :
4 g d'un catalyseur à 5 % de ruthénium (cf. exemple 3)
22,8 g (0,2 moles) de TFA

On élimine l'oxygène et l'azote comme il est dit à l'exemple 1 puis on pressurise sous 40 bars d'hydrogène. On chauffe à 150 °C.

La pression s'élève à 45 bars à 150 °C. On la maintient constante pendant toute la durée de la réaction.

On effectue une purge continue du ciel gazeux comme il est dit à l'exemple 1. Cette purge représente environ 10 % de la quantité totale d'hydrogène nécessaire à l'accomplissement de la réaction.

La réaction stoppe après 1 heure. Le taux de transformation du TFA est de 100 %. Le rendement en TFE est de 95,5 %. Il s'est aussi formé 3,5 % de $CF_3CH_3$ ; 0,6 % d'éthane et 0,4 % de méthane.

Exemple 6

On répète l'exemple 5 dans le même réacteur avec les charges suivantes :

| | |
|---|---|
| Ru/C à 5 % de Ru | 2,5 g |
| Eau | 7,5 g |
| TFA | 22,8 g (0,2 moles) |

L'autoclave est pressurisé avec 50 bars d'hydrogène et on chauffe à 90 °C. On maintient la pression constante égale à 50 bars et on purge en continu environ 10 % du débit d'hydrogène alimenté.

La réaction stoppe après 4 heures et 10 minutes.

Le taux de transformation du TFA est de 100 %.

Le rendement (RT) en TFE est de 92 %. Il y a en complément :

| | |
|---|---|
| $CF_3CH_3$ | 7,2 % |
| $C_2H_6$ | 0,5 % |
| $CH_4$ | 0,2 % |

Exemple 7

On opère comme dans l'exemple 5 avec les charges suivantes :

| | |
|---|---|
| oxyde de ruthénium (non supporté) | 2 g |
| Eau | 6 ml |
| acide trifluoroacétique | 22,8 g (0,2 mole) |

On conduit la réaction à 150 °C sous 50 bars de pression totale en purgeant en continu environ 12 % de la quantité totale d'hydrogène alimenté.

La réaction stoppe après 1 heure 40 minutes.

Le taux de transformation de l'acide trifluoroacétique est de 100 % ; le rendement (RT) en TFE est de 86,9 %. Celui de $CF_3CH_3$ de 9,6 %. Il y a en outre 2,7 % d'éthane et 0,85 % de méthane.

Exemple 8

On opère comme dans l'exemple 7 en remplaçant le catalyseur à base de ruthénium par un catalyseur à base d'iridium déposé à 5 % sur un charbon de grande surface spécifique (> 900 $m^2/g$).

Les charges sont :

| | |
|---|---|
| catalyseur Iridium à 5 %/charbon | 4 g |
| Eau | 8 ml |
| TFA | 0,2 mole |

La réaction est conduite à 150 °C sous 45 bars de pression totale.

On purge en continu une quantité de ciel gazeux égale à 3 $lh^{-1}$ (TPN) ce qui représente environ 30 % de la quantité totale d'hydrogène nécessaire à la réaction.

La réaction stoppe après 1 heure.

Le taux de transformation du TFA est de 100 %. Le rendement en TFE est de 83,5 %, celui du trifluoroéthane de 16 %. Il y a en outre 0,35 % de méthane et 0,25 % d'éthane.

Exemple 9

On répète l'exemple 8 mais à 150 °C sous 10 bars de pression totale.

La réaction stoppe après 3 heures.

Le taux de transformation du TFA est de 70 %.

Le rendement (RT) en trifluoroéthanol (TFE) s'élève à 96 %. Il s'est formé en outre 3,7 % de $CF_3CH_3$ ; 0,2 % d'éthane et 0,1 % de $CH_4$.

Exemple 10

On charge dans le même réacteur que celui utilisé dans les exemples précédents :

4 g d'un catalyseur à 5 % de Pt déposé sur charbon de surface spécifique supérieure à 900 m²/g

8 ml d'eau

22,8 g d'acide trifluoroacétique soit 0,2 mole.

On hydrogène à 180 °C sous 40 bars de pression totale. On purge en continu une partie du ciel gazeux correspondant à 10 % environ de la quantité totale d'hydrogène nécessaire à l'accomplissement de la réaction.

La réaction s'arrête au bout de 2 h 45 minutes.

Le taux de transformation de l'acide trifluoroacétique est de 94 %. Le rendement en trifluoroéthanol est de 96 %. Il s'est ainsi formé :

2,5 % de $CF_3CH_3$

1,1 % de $C_2H_6$

0,4 % de $CH_4$.

**Revendications**

1. Procédé de préparation de trifluoroéthanol par hydrogénation en phase liquide d'acide trifluoroacétique caractérisé en ce qu'on opère en présence d'un catalyseur à base de ruthénium, rhodium, iridium ou platine, sous une pression totale comprise entre 5 et 50 bars, avec un rapport du volume de la phase liquide mise en œuvre dans le réacteur au volume total du réacteur supérieur à 1/2 environ et en ce que l'on maintient pendant la réaction une pression partielle d'hydrogène supérieure à 1 bar absolu en laissant échapper en continu au cours de la réaction une partie de la phase gazeuse présente dans le réacteur.

2. Procédé selon la revendication 1, caractérisé en ce que la pression totale est comprise entre 5 et 30 bars.

3. Procédé selon l'une quelconque des revendications précédentes caractérisé en ce que le rapport du volume de la phase liquide au volume total du réacteur est compris entre 0,5 et 0,8.

4. Procédé selon l'une quelconque des revendications précédentes, caractérisé en ce que la pression partielle d'hydrogène est supérieure à 5 bars.

5. Procédé selon l'une quelconque des revendications précédentes, caractérisé en ce que le métal catalyseur est mis en œuvre sous forme métallique, d'un oxyde ou d'un sel ou d'un mélange d'entre eux.

6. Procédé selon la revendication 5, caractérisé en ce que le catalyseur est déposé sur un support choisi parmi le charbon, la silice et l'alumine.

7. Procédé selon l'une quelconque des revendications précédentes, caractérisé en ce que l'on met en œuvre moins de 10 % en poids de catalyseur exprimés en métal par rapport à l'acide trifluoroacétique.

**Claims**

1. Process for the preparation of trifluoroethanol by liquid phase hydrogenation of trifluoroacetic acid, characterized in that it is performed in the presence of a catalyst based on ruthenium, rhodium, iridium or platinum, under a total pressure of between 5 and 50 bars, with a ratio of the volume of the liquid phase employed in the reactor to the total volume of the reactor greater than approximately 1/2 and in that a partial pressure of hydrogen which is greater than 1 bar absolute is maintained during the reaction by allowing a part of the gaseous phase present in the reactor to escape continuously during the reaction.

2. Process according to Claim 1, characterized in that the total pressure is between 5 and 30 bars.

3. Process according to either of the preceding claims, characterized in that the ratio of the volume of the liquid phase to the total volume of the reactor is between 0.5 and 0.8.

4. Process according to any one of the preceding claims, characterized in that the partial pressure of hydrogen is greater than 5 bars.

5. Process according to any one of the preceding claims, characterized in that the catalyst metal is used in metallic form, in the form of an oxide or a salt, or of a mixture thereof.

6. Process according to Claim 5, characterized in that the catalyst is deposited on a support chosen from charcoal, silica and alumina.

7. Process according to any one of the preceding claims, characterized in that less than 10 % by weight of catalyst, expressed as metal is used, based on the trifluoroacetic acid.

**Patentansprüche**

1. Verfahren zur Herstellung von Trifluorethanol durch Hydrogenierung von Trifluoressigsäure in flüssiger Phase, dadurch gekennzeichnet, daß man in Gegenwart eines Katalysators auf Basis von Ruthenium, Rhodium, Iridium oder Platin arbeitet, bei einem Gesamtdruck zwischen 5 und 50 bar, mit einem Volumenverhältnis von im Reaktor umgesetzter flüssiger Phase zum Gesamtvolumen des Reaktors größer als ungefähr 1/2 und daß man während der Reaktion einen Partialdruck an Wasserstoff von größer als absolut 1 bar aufrechterhält, indem man kontinuierlich im Verlauf der Reaktion einen Teil der im Reaktor anwesenden Gasphase abläßt.

2. Verfahren nach Anspruch 1, dadurch gekennzeichnet, daß der Gesamtdruck zwischen 5 und 30 bar liegt.

3. Verfahren nach einem der vorhergehenden Ansprüche, dadurch gekennzeichnet, daß das Verhältnis des Volumens der flüssigen Phase zum Gesamtvolumen des Reaktors zwischen 0,5 und 0,8 liegt.

4. Verfahren nach einem der vorhergehenden Ansprüche, dadurch gekennzeichnet, daß der Partialdruck an Wasserstoff größer ist als 5 bar.

5. Verfahren nach einem der vorhergehenden Ansprüche, dadurch gekennzeichnet, daß der Metallkatalysator in metallischer Form, in Form eines Oxids oder eines Salzes oder einer Mischung von diesen eingesetzt wird.

6. Verfahren nach Anspruch 5, dadurch gekennzeichnet, daß der Katalysator auf einen Träger aufgebracht wird, der ausgewählt wird zwischen Kohle, Siliciumoxid und Aluminiumoxid.

7. Verfahren nach einem der vorhergehenden Ansprüche, dadurch gekennzeichnet, daß man weniger als 10 Gew.-% des Katalysators bezogen auf das Verhältnis von Metall zu Trifluoressigsäure einsetzt.